# EUROPÄISCHE PATENTSCHRIFT

(11) **EP 2 308 403 B1**
(45) Veröffentlichungstag und Bekanntmachung des Hinweises auf die Patenterteilung: **27.03.2013**
(21) Anmeldenummer: 10013345.3
(22) Anmeldetag: 06.10.2010
(51) Int. Cl.: A61B 17/80, A61B 17/86

(54) **Knochenschraube sowie System**
Bone screw and system
Vis à os et système

(30) Priorität: 12.10.2009 DE 102009049168
(43) Veröffentlichungstag der Anmeldung: 13.04.2011
(73) Patentinhaber: NORMED Medizin-Technik GmbH, 78532 Tuttingen (DE)
(72) Erfinder: Pech, Uwe, 78532 Tuttlingen (DE); Wörner, Roland, 78532 Tuttlingen (DE)
(74) Vertreter: Wagner, Kilian

(56) Entgegenhaltungen:
- DE-U1-202005 014 850
- US-A1- 2006 009 771
- US-A1- 2007 162 018
- US-A1- 2008 140 130

## Beschreibung

Die Erfindung betrifft eine Knochenschraube mit einem ein Knochengewinde aufweisenden Schaft und mit einem axial benachbart zum Knochengewinde angeordneten Gewinde. Ferner betrifft die Erfindung ein System, umfassend eine Knochenplatte sowie eine Knochenschraube zum Zusammenwirken mit einer Durchgangsöffnung der Knochenplatte.

Heute werden in der Chirurgie sogenannte winkelstabile Fixationssysteme eingesetzt umfassend eine Knochenplatte mit mindestens einem Innengewindeloch sowie eine Knochenschraube, die in verschiedenen Winkeln in das Innengewindeloch einschraubbar und mit der Knochenplatte verblockbar ist.

Ein derartiges Fixationsprinzip ist beispielsweise aus der EP 114 3867 B1 bekannt. Das bekannte Fixationssystem beruht auf der Idee, beim Einschrauben der Knochenschraube durch Materialumformung eine Gewindeverbindung zwischen Knochenschraube und Knochenplatte zu erzeugen. Nachteilig bei dem bekannten Fixationssytem ist zum einen, dass die Einschraubtiefe der Knochenschraube nicht definiert ist, bzw. dass nicht beeinflussbar ist, ab welcher Einschraubtiefe es zu einer Verblockung durch Kaltverschweißung kommt. Kommt das Kalterverschweißen zu früh, ist die Knochenplatte noch nicht stabil genug gesichert. Kommt das Kaltverschweißen zu spät, ist die Winkelstabilität nicht sichergestellt. Ferner von Nachteil ist, dass sich die mit der Knochenplatte kaltverschweißte Knochenschraube nur sehr schwer wieder lösen lässt. Häufig kommt es hierbei zum Abreißen der Knochenschraube.

Aus der WO 2004/086990 A1 ist alternatives Fixationssystem bekannt, bei dem der Schraubenkopf nicht mit einem umlaufenden Verblockungsgewinde versehen ist, sondern bei dem der Verblockungsgewindeverlauf durch drei gleichmäßig, in Umfangsrichtung beabstandete Abflachungen unterbrochen ist.

Nachteilig bei dem bekannten Fixationssystem ist die aufwändige Knochenschraubengeometrie und die Tatsache, dass die Eindringtiefe der Knochenschraube in die Knochenplatte nicht definiert ist.

Aus der US 2006/0009771 A1 ist eine Knochenschraube bekannt. Bei dem dort in Fig. 7 gezeigten Ausführungsbeispiel ist von einem Kopfgewinde axial in Richtung Kopfende ein Konusabschnitt beabstandet und zwar über einen Zylinderabschnitt. Das Kopfgewinde hat eine zylindrische Hüllkontur. Mit der bekannten Knochenschraube können Kaltverschweißungseffekte nicht sicher verhindert werden, was als nachteilig empfunden wird. Aus derselben Druckschrift ist in Fig. 8c eine alternative Knochenschraube bekannt, bei der mit Axialabstand zu einem Kopfgewinde nicht eine Konusfläche, sondern ein konvex gewölbter Abschnitt über ein Zylinderabschnitt beabstandet ist. Auch hier können Kaltverschweißungseffekte nicht sicher vermieden werden.

Eine alternative Knochenschraube ist aus der US 2005/0070904 A1 bekannt. Diese umfasst ein Knochengewinde und ein Kopfgewinde, die axial voneinander beabstandet sind. Eine kopfseitige Konusfläche ist über einen Zylinderabschnitt von dem Kopfgewinde beabstandet angeordnet.

Auch aus der FR 2 910 800 A1 ist eine Knochenschraube mit einem Kopfgewinde und einem Knochengewinde bekannt, wobei das Kopfgewinde von einer Kopf-Konusfläche (Flanke) über einen Zylinderabschnitt beabstandet ist. Auch hier kann eine Kaltverschweißung zwischen Knochenschraube und Knochenplatte nicht sicher vermieden werden.

Aus der US 2007/162018 A1 ist eine gattungsbildende Knochenschraube bekannt, die hinsichtlich ihrer Verblockungsneigung verbesserungsbedürftig ist.

Aus der US 2006/009771 A1 ist eine Knochenschraube bekannt, deren Kopfgewinde ein Freistich zugeordnet ist, der von einer oberen Zweistichflanke mit geringer Radialerstreckung begrenzt ist. Die Hüllkontur des Kopfgewindes ist zylindrisch. Die Schraube ist hinsichtlich ihrer Verblockungsneigung verbesserungsbedürftig.

Die US 2008/140130 A1 zeigt eine Knochenschraube bei der das Kopfgewinde eine konische Hüllkontur aufweist

Die DE 20 2005 014850 U1 zeigt eine Knochenschraube bei der die Hüllkontur des Kopfgewindes kegelig, kalottenförmig, konkav oder konvex ausgeführt sein kann. Auch diese Knochenschraube ist hinsichtlich ihres Verblockungsverhaltens verbesserungsbedürftig.

Ausgehend von dem vorgenannten Stand der Technik liegt der Erfindung die Aufgabe zugrunde, eine Knochenschraube für ein winkelstabiles (Fixations-) System anzugeben, die derart ausgebildet ist, dass die Eindringtiefe der Knochenschraube bzw. die Axialposition, in der die Knochenschraube mit der ihr zugeordneten Knochenplatte verblockt, definiert ist. Bevorzugt soll eine Kaltverschweißung zwischen Knochenschraube und Knochenplatte zum Sicherstellen eines erleichterten Lösens der Knochenschraube siche vermieden werden. Ferner besteht die Aufgabe darin, ein entsprechend optimiertes (Fixations-)System anzugeben.

Diese Aufgabe wird hinsichtlich der Knochenschraube mit den Merkmalen des Anspruchs 1 und hinsichtlich des Systems mit den Merkmalen des Anspruchs 7 gelöst.

Vorteilhafte Weiterbildungen der Erfindungen sind in den Unteransprüchen angegeben.

Die Erfindung versucht das im Stand der Technik auftretende Kaltverschweißen bzw. "Fressen" dadurch zu verhindern, dass die Knochenschraube beim Verblocken definiert mit einer das Gewinde nach oben begrenzenden, umlaufenden Freistichflanke eines Freistichs erfolgt, der in einer quer zur Längsmittelachse der Knochenschraube angeordneten gedachten Ebene liegt. Anders ausgedrückt endet das oberhalb des, insbesondere genormten, Knochengewindes angeordnete Gewinde bei einer nach dem Konzept der Erfindung ausgebildeten Knochenschraube mit Axialabstand vor der Knochenschraubenoberseite, wobei das Gewinde freigestochen ist, bzw. ein Freistich gebildet ist, dessen obere, als Schrägebene ausgebildete Freistichfläche bzw. Flanke für die Verblockung mit der Knochenplatte verantwortlich ist. Bei dem Freistich handelt es sich im Prinzip um eine Gewinderille ohne Steigung. Durch das Vorsehen der Freistichfläche, die bevorzugt die Hüllkontur des Gewindes nach radial außen überragt, dockt die Knochenschraube lediglich mit der Freistichflanke an einer Innengewindeflanke einer Innengewindedurchgangsöffnung in der Knochenplatte oder an einer Innengewindekante der Innengewindedurchgangsöffnung oder am oberen Umfangsrand der Durchgangsöffnung der Knochenplatte (in Abhängigkeit des Einbringungswinkels) an. Ein Kaltverschweißen wird dadurch verhindert, dass aufgrund des Vorsehens der Freistichflanke quasi unmittelbar nach Auftreffen der Freistichflanke auf die Knochenplatte eine Verblockung erfolgt und dass nach dem Auftreffen nur noch eine minimal Axialverstellung, von vielleicht 0,2 Millimetern oder weniger, möglich ist - dies reicht nicht für einen Kaltverschweißvorgang aus. Ein weiterer wesentlicher Vorteil einer zum Konzept der Erfindung ausgebildeten Knochenschraube besteht darin, dass die Freistichflanke die Eindringtiefe begrenzt, da nach Auftreffen der Freistichflanke auf die Knochenschraube, wie zuvor erläutert, quasi keine Axialverstellung mehr möglich ist. Somit wird also ein Verblocken immer an einer definierten Achse, die sich lediglich geringfügig in Abhängigkeit des Einbringungswinkels ändert, erreicht.

Die Freistichflanke erstreckt sich erfindungsgemäß bis zum Auslauf des Gewinderillengrundes des in den Freistich auslaufenden Gewindes. Anders ausgedrückt grenzt die Freistichflanke unmittelbar an das Ende des benachbart zum Knochengewinde angeordneten Gewindes an, vorzugsweise an den Gewinderillengrund der Gewinderille. Es wird also erfindungsgemäß auf einen beispielsweise zylindrischen Abstandsabschnitt zwischen Gewinde und Freistichflanke verzichtet.

Ferner ist bei der erfindungsgemäßen Knochenschraube vorgesehen, dass das Knochengewinde und das bevorzugt unterschiedlich zu dem Knochengewinde ausgebildete Gewinde die gleiche Steigung aufweisen. Ganz besonders bevorzugt beträgt diese, zumindest näherungsweise 0,5.

Besonders zweckmäßig ist das erfindungsgemäße Merkmal, nach welchem das benachbart zum Knochengewinde angeordnete Gewinde radial weiter nach außen ragt, also in einem Schraubenkopf eingebracht ist, der eine größere Radialerstreckung aufweist, als der, vorzugsweise eine zylindrische Hüllkontur aufweisende Schaft der Knochenschraube.

Besonders hervorzuheben ist ein gemäß der Erfindung vorgesehenes Merkmal, nachdem der das Gewinde aufweisende Abschnitt des Schraubenkopfes eine konische Hüllkontur aufweist, also eine Hüllkontur, deren Durchmesser, in axialer Richtung betrachtet, hin zum Schaft abnimmt. Der Konusabschnitt ermöglicht ein Verkippen der Knochenschraube in einer, vorzugsweise mit einem Innengewinde versehenen Durchgangsöffnung in der Knochenplatte.

Ganz besonders bevorzugt ist der Winkel α zwischen der Längsmittelachse der Knochenschraube und einer Konusmantelfläche des Konusabschnittes aus einem Wertebereich zwischen etwa 5° und etwa 30°, ganz besonders bevorzugt zwischen etwa 10° und etwa 20° gewählt. Noch weiter bevorzugt beträgt dieser (halbe) Konuswinkel, etwa 15°. Die Wahl des Konuswinkels entscheidet über den maximal möglichen Verkippungswinkel und damit über die Größe der Mulitidirektionalität eines die Knochenschraube umfassenden Fixationssystems.

In Weiterbildung der Erfindung ist mit Vorteil vorgesehen, dass ein Winkel α zwischen einer quer zur Längsmittelachse der Knochenschraube angeordneten Ebene (Freistichebene), innerhalb der der Freistichgrund liegt und der für die Verblockung verantwortlichen Freistichflanke aus einem Winkelbereich zwischen etwa 10° und etwa 50°, vorzugsweise zwischen etwa 15° und etwa 45°, bevorzugt zwischen etwa 20° und etwa 35° gewählt ist. Ganz besonders bevorzugt beträgt dieser Winkel etwa 30°.

Ganz besonders bevorzugt handelt es sich bei der Freistichflanke um eine linear ansteigende Ebene, das heißt, um eine konzentrisch zur Längsmittelachse der Knochenschraube angeordnete, nicht gewölbte Schrägebene.

Besonders zweckmäßig ist es, wenn das, vorzugsweise in dem vorerwähnten Konusabschnitt angeordnete, axial benachbart zum Knochengewinde platzierte Gewinde auf einem gedachten Zylinder sitzt, anders ausgedrückt, enden die Gewinderillen des Gewindes bevorzugt radial innen auf einer gedachten Zylindermantelfläche bzw. bilden eine gedachte Schraubenlinie auf dieser Zylindermantelfläche, sodass die Gewinderillentiefe aufgrund der Konusform des das Gewinde aufnehmenden Konusabschnittes mit zunehmendem Abstand vom Knochengewinde zunimmt. Bei einer alternativen Ausführungsform enden die Gewinderillen auf einer gedachten Konusmantelfläche, wodurch die Verkippneigung der Schraube erhöht werden kann.

Besonders zweckmäßig erscheint es, wenn das Gewinde (Kopfgewinde) axial von dem Knochengewinde beabstandet angeordnet ist, also das Knochengewinde mit Abstand zu dem Gewinde endet. Bevorzugt sind das Knochengewinde und das Gewinde derart relativ Umfangsrichtung zueinander positioniert bzw. ist der Axialabstand, der Auslauf des Knochengewindes und der Einlauf des Gewindes derart relativ zueinander positioniert, dass es eine gedachte Schraubenlinie einheitlicher Steigung gibt, zu der die Gewinderillen sowohl des Gewindes als auch des Knochengewindes in radialer Richtung fluchten.

Im Hinblick auf die Ausgestaltung des Gewindes gibt es unterschiedliche Möglichkeiten. Besonders bevorzugt handelt es sich um ein mehrgängiges, insbesondere dreigängiges Gewinde. Besonders zweckmäßig ist es, das Gewinde als metrisches Gewinde auszubilden. Unabhängig von der konkreten Ausgestaltung des Gewindes sollte das Gewinde als in Umfangsrichtung nicht unterbrochenes, d. h. umlaufendes Gewinde ausgebildet sein. Bei dem Gewinde handelt es sich bevorzugt nicht um ein Knochengewinde sondern vorzugsweise um ein zum Verschrauben mit einem Innengewinde ausgebildetes Außengewinde.

Die Erfindung führt auch auf ein (Fixations-)System mit einer wie zuvor beschriebenen ausgebildeten Knochenschraube und einer Knochenplatte mit mindestens einer Durchgangsöffnung, die zum Zusammenwirken mit der Knochenschraube ausgebildet ist. Bevorzugt umfasst die Durchgangsöffnung ein Innengewinde zum Zusammenwirken mit dem Gewinde der Knochenschraube.

Bevorzugt weist das Knochenplatteninnengewinde, insbesondere exakt, die gleiche Steigung und Parallelität auf wie das Gewinde (insbesondere das Schraubenkopfgewinde) der Knochenschraube. Vorzugsweise sind Durchmesser und Steigung der vorgenannten Gewinde identisch und liegen innerhalb der in den Normen gegebenen Toleranzen. Bevorzugt hängen der Durchmesser und die Steigung von der jeweiligen Schraubenkopfgröße ab. Bevorzugt wird das Knochengewinde der Schraube dem Kopfgewinde angepasst, wobei Knochengewinde und Kopfgewinde bevorzugt die gleiche Steigung aufweisen. Vorzugsweise existieren Unterschiede nur in der Form und im Durchmesser der beiden Gewinde der Knochenschraube. Durch die identischen Steigungen wird ein "Verklemmen" verhindert und ein definiertes Blockieren sichergestellt.

Die Durchgangsöffnung bzw. das ggf. vorgesehene Innengewinde der Durchgangsöffnung ist bevorzugt derart auf die Knochenschraube abgestimmt, dass die Knochenschraube zum einen nicht durch die Durchgangsöffnung vollständig durchgeführt werden kann und derart, dass unabhängig von dem Einbringungswinkel der Knochenschraube in die Durchgangsöffnung eine Verblockung zwischen der Freistichflanke der Knochenschraube und der Durchgangsöffnung der Knochenplatte erreichbar ist.

Besonders zweckmäßig ist es, wenn die Knochenschraube aus einem härteren Material als die Knochenplatte ausgebildet ist, um eine Deformation der Knochenschraube beim Verblockungsvorgang ausschließen zu können und um die Knochenplatte, bei entsprechender Dickenwahl verformen und an die anatomischen Verhältnisse anpassen zu können.

Besonders zweckmäßig ist es, wenn sich die schräg im Bezug auf eine quer zur Längsmittelachse der Knochenschraube verlaufende Freistichkante mit ihrem oberen Umfangsrand einen größeren Durchmesser aufweist, als der Kerndurchmesser, das heißt der innerste Durchmesser der Durchgangsöffnung der Knochenschraube, um ein Hindurchrutschen zu vermeiden.

Weitere Vorteile, Merkmale und Einzelheiten der Erfindung entnehmen Sie der nachfolgenden Beschreibung bevorzugter Ausführungsbeispiele sowie anhand der Zeichnungen.

Diese zeigen Ihnen:
- Fig.1:: eine Darstellung einer möglichen Ausführungsform einer Knochenschraube,
- Fig. 2:: ein vergrößertes Detail einer besonders bevorzugten Ausführungsvariante einer Knochenschraube,
- Fig. 3 u. 4:: unterschiedliche Herstellungsstadien einer noch nicht fertigen Knochenschraube,
- Fig. 5:: eine ausschnittsweise Darstellung eines Systems, umfassend eine Knochenplatte und eine winklig eingebrachte, verblockte Knochenschraube und
- Fig. 6:: ein System, umfassend eine Knochenplatte und eine Knochenschraube, wobei die Knochenschraube parallel zur Längsmittelachse einer Durchgangsöffnung der Knochenplatte in die Durchgangsöffnung eingebracht ist.

In den Figuren sind gleiche Elemente und Elemente mit der gleichen Funktion mit den gleichen Bezugszeichen gekennzeichnet.

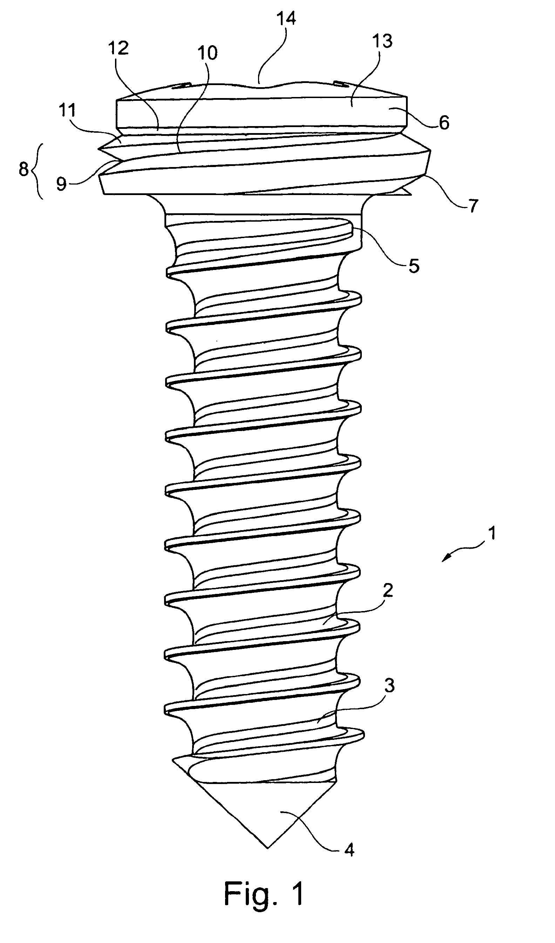

In Fig. 1 ist eine Ausführungsvariante einer nach dem Konzept der Erfindung ausgebildeten Knochenschraube 1 gezeigt. Diese umfasst einen langgestreckten Schaft 2 mit einer zylindrischen Hüllkontur, wobei in den Schaft 2 ein genormtes Knochengewinde 3 eingebracht ist. Die Knochenschraube 1 bzw. der Schaft 2 endet an seinem vorderen (unteren) Ende mit einer konischen Spitze 4. Mit seinem von der Spitze 4 abgewandten Ende 5 endet das Knochengewinde 3 unterhalb eines Schraubenkopfes 6, der den Schaft 2 in radialer Richtung nach außen überragt. Das Knochengewinde 3 endet mit Axialabstand zu einem im Schraubenkopf 6 vorgesehenen (hier metrischen) Gewinde 7, welches in einen Axialabschnitt 8 des Schraubenkopfes 6 eingebracht ist, der eine konische Hüllkontur (nicht eingezeichnet) aufweist. Das Gewinde 7 bzw. der Gewinderillengrund 9 der Gewinderille 10 des Gewindes 7 schraubt sich entlang eines gedachten Zylinders bzw. endet radial innen auf einer gedachten Zylindermantelfläche mit der Folge, dass die Tiefenerstreckung der Gewinderille 10 in radialer Richtung mit kleiner werdendem Abstand zum Knochengewinde 3 abnimmt. Das Gewinde 7 läuft aus bzw. endet in einem umlaufenden Freistich 11, also einer Gewinderille ohne Steigung, die nach oben von einer als Schrägebene ausgebildeten Freistichflanke 12 begrenzt ist.

Oberhalb des Gewindes 7, welches in dem mit Axialabstand zum oberen Ende der Knochenschraube 1 angeordneten Freistich 11 endet, ist ein durchmesserreduzierter, zylindrisch konturierter Endabschnitt 13 des Kopfes angeordnet, in den stirnseitig ein, beispielsweise als Torxantrieb ausgebildeter Antrieb 14 eingebracht ist.

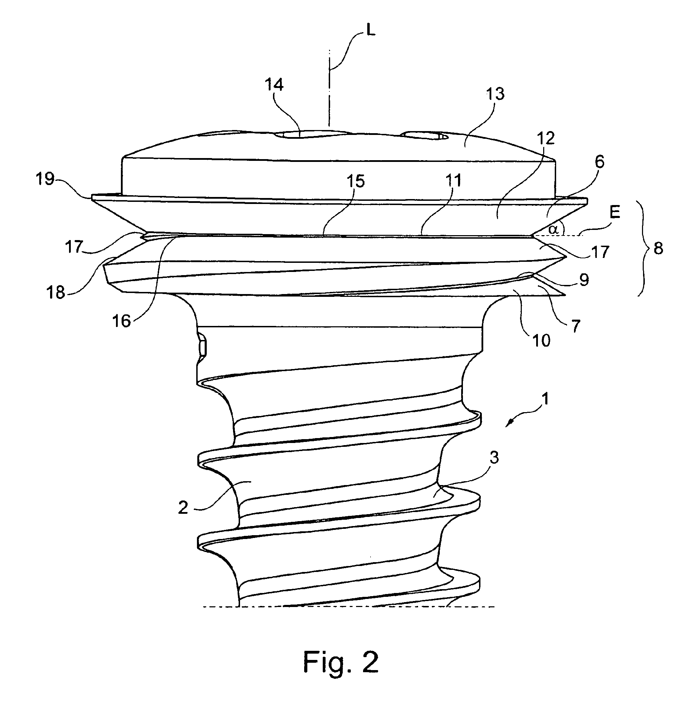

Fig. 2 zeigt ein bevorzugtes Ausführungsbeispiel einer Knochenschraube 1 ausschnittsweise genauer. Zu erkennen ist das Knochengewinde 3 im Schaft 2 sowie der Schraubenkopf 6 mit seinem eine sich konisch verjüngende Hüllkontur aufweisenden Axialabschnitt 8, in welchem das sich von dem Knochengewinde 3 unterscheidende Gewinde 7 (Kopfgewinde) eingebracht ist. Dieses weist eine schraubenförmig verlaufende Gewinderille 10 mit einem Gewinderillengrund 9 (tiefste Gewindelinie) auf. Zu erkennen ist, dass der Gewinderillengrund 9 in einen Freistichgrund 15 übergeht. Der Freistichgrund 15 und der Gewinderillengrund 9 liegen im Kreuzungs- bzw. Einmündungspunkt 16 auf derselben gedachten Durchmesserlinie. Zu erkennen ist, dass der Freistich 11 eine konzentrisch zur Längsmittelachse L der Schraube angeordnete, obere Freistichflanke 12 aufweist, die als Schrägebene ausgebildet ist, die bis zum Freistichgrund 15 reicht. Auf der gegenüberliegenden Seite wird der Freistich 11 begrenzt von einer unteren Freistichflanke 17, in die eine Gewindeflanke 18 des Gewindes 7 im Bereich des Einmündungspunktes 16 übergeht. Die Freistichflanken 12, 17 begrenzen im Längsschnitt betrachtet eine freie Dreieckfläche. Aus Fig. 2 ergibt sich, dass der nach radial innen abgesetzte Endabschnitt 13 in radialer Richtung nach innen versetzt ist zum äußersten, oberen Umfangsrand 19 der oberen Freistichflanke 12.

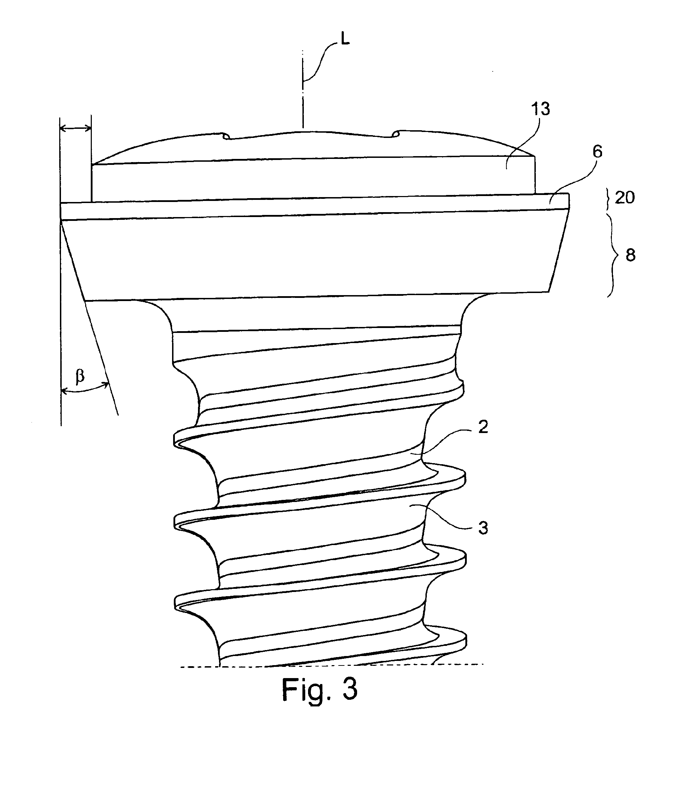

Ein Winkel α zwischen einer sich quer zur Längsmittelachse L erstreckenden Freistichebene E und der Freistichflanke 12 beträgt in dem gezeigten Ausführungsbeispiel etwa 30°. Der nicht eingezeichnete Winkel zwischen der Mantelfläche der konischen Hüllkontur des Axialabschnittes 8 und der Längsmittelachse L beträgt in dem gezeigten Ausführungsbeispiel etwa15° (vgl. Fig. 3 = Winkel β).

In Fig. 3 ist eine noch nicht fertige Knochenschraube 1 gezeigt. Zu erkennen ist bereits der Schaft 2 mit Knochengewinde 3 und der Schraubenkopf 6 mit seinem Konusabschnitt 8 (Axialabschnitt), in den in einem nächsten Fertigungsschritt das in Fig. 2 gezeigte Gewinde, beispielsweise durch Drehen, eingebracht wird. Zu erkennen ist, dass axial oben an den konischen Axialabschnitt 8 ein Zylinderabschnitt 20 mit kurzer Axialerstreckung angrenzt, zu dem radial abgesetzt der zylindrisch konturierte Endabschnitt 13 angeordnet ist.

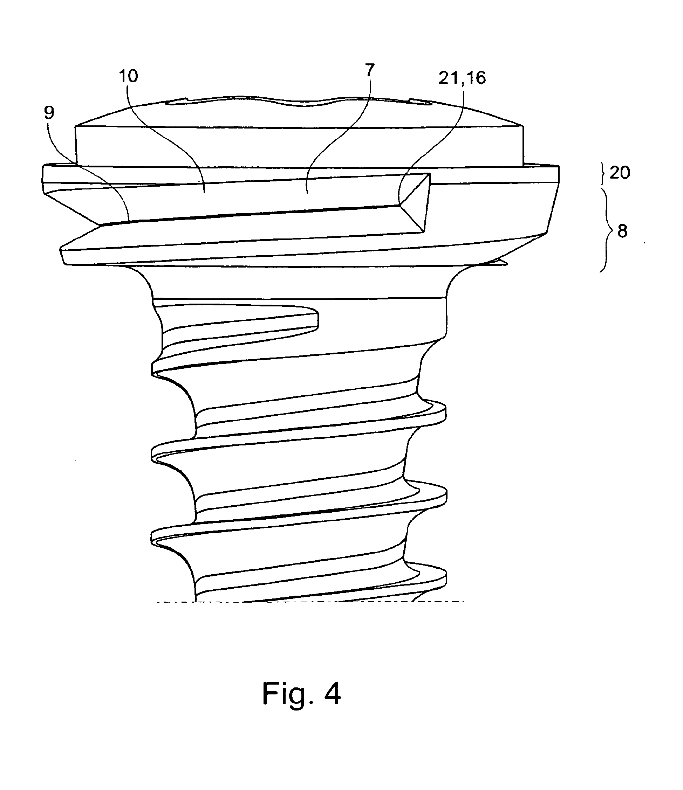

Fig. 4 zeigt den nächsten Fertigungsschritt, mit Hilfe dessen bereits das Gewinde 7 in den Axialabschnitt 8 und ein Stück weit auch in den Zylinderabschnitt 20 eingebracht wurde, vorzugsweise durch Drehen. Erkennbar ist insbesondere die Gewinderille 10 mit Gewinderillengrund 9. Die Gewinderille 10 endet vor Erreichen des oberen axialen Endes des zylindrischen Axialabschnittes 20. Der Gewinderillengrund 9 endet mit Axialabstand vor dem oberen Ende des konischen Axialabschnittes 8. In einem nächsten Fertigungsschritt wird der aus Fig. 2 ersichtliche Freistich 11 eingebracht, derart, dass der Freistichgrund 15 auf der axialen Höhe des Endes 21 des Gewinderillengrundes 9 zum Liegen kommt. Das Ende 21 fällt mit dem späteren Einlaufpunkt 16 zusammen.

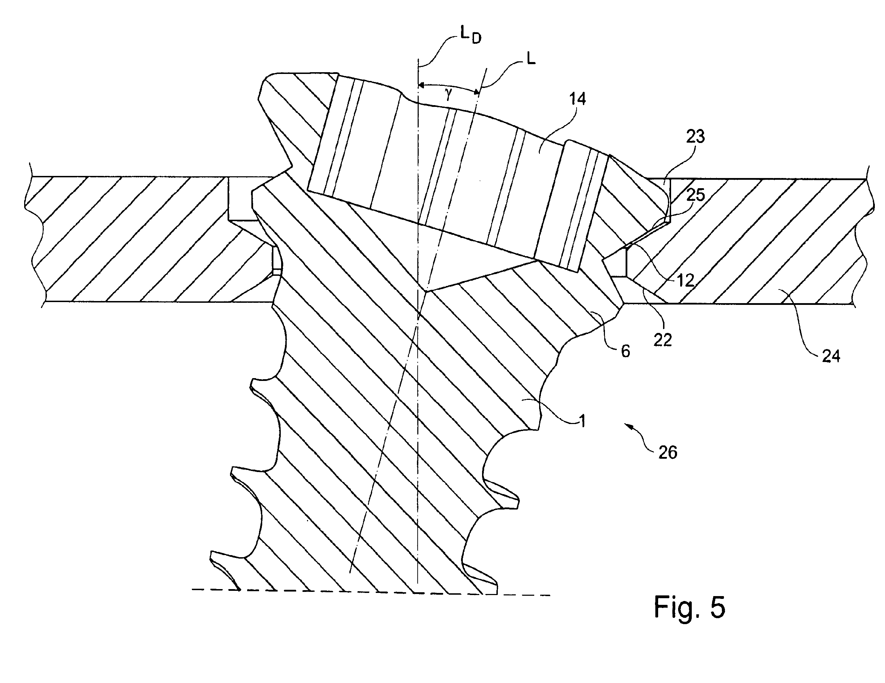

Fig. 5 zeigt eine Knochenschraube 1, die bevorzugt wie in Fig. 2 ausgebildet ist, innerhalb einer mit Innengewinde 22 versehenen Durchgangsöffnung 23 in einer Knochenplatte 24. Sowohl die Knochenplatte 24 als auch die Knochenschraube 1, die gemeinsam ein (Fixations-)System 26 bilden, sind auch aus Titan hergestellt. Die Längsmittelachse L der Knochenschraube 1 schließt mit einer Längsmittelachse LD der Durchgangsöffnung 23 einen Winkel Y ein, der in dem gezeigten Ausführungsbeispiel 15° beträgt. Die obere Freistichflanke 12 stützt sich in der Zeichnungsebene rechts auf einer Innengewindeflanke 25 ab und ist mit dieser verblockt, sodass die Knochenschraube 1 winkelstabil unter dem Winkel Y gehalten und die Knochenplatte 24 sicher fixiert ist. Auf der dem Anlagepunkt der Freistichflanke 12 diametral und schräg gegenüberliegenden Seite stützt sich die Knochenschraube 1 mit einer Unterseite des Schraubenkopfes 6 an der inneren Kante der Innengewindeflanke 25 an der Knochenplatte 24 ab.

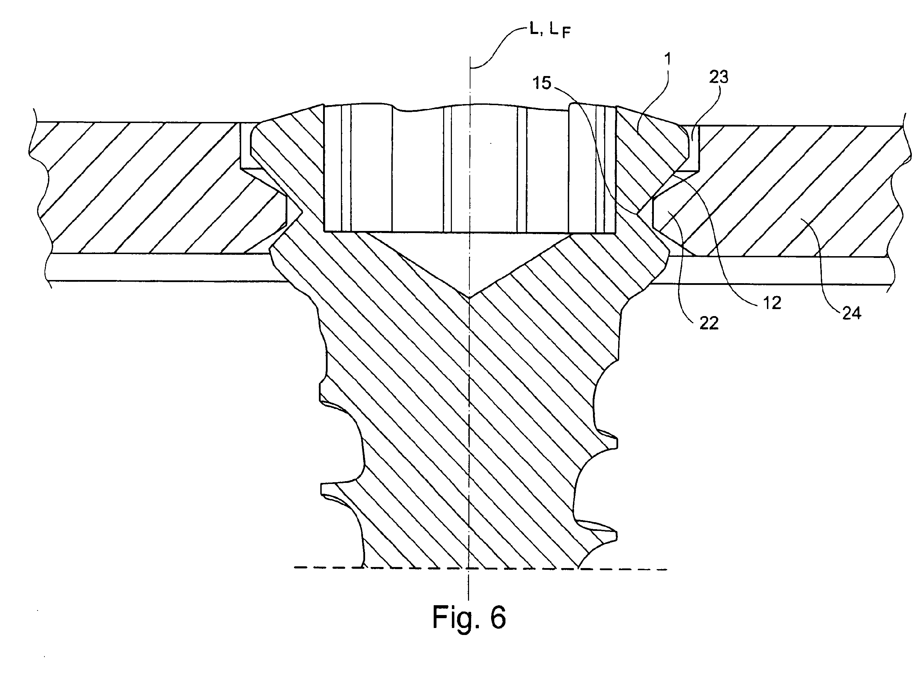

Bei dem in Fig. 6 gezeigten System 26 fallen die Längsmittelachse L der Knochenschraube 1 und die Längsmittelachse L_{F} der Durchgangsöffnung 23 in der Knochenplatte 24 zusammen. Zu erkennen ist, dass der Freistichgrund 15 mit Radialabstand zum Innengewinde 22 angeordnet ist und sich die Knochenschraube 1 mit der oberen Freistichflanke 12 axial an einer inneren oberen Kante des Innengewindes 22 abstützt. Aufgrund des Radialabstandes zwischen Freistichgrund 15 und Innengewinde 22 ist die Reibung reduziert.

### Bezugszeichen

- **1**: Knochenschraube
- **2**: Schaft
- **3**: Knochengewinde
- **4**: Spitze
- **5**: Ende
- **6**: Schraubenkopf
- **7**: Gewinde
- **8**: konischer Axialabschnitt
- **9**: Gewinderillengrund
- **10**: Gewinderille
- **11**: Freistich
- **12**: obere Freistichflanke
- **13**: Endabschnitt
- **14**: Antrieb
- **15**: Freistichgrund
- **16**: Einmündungspunkt
- **17**: Freistichflanke
- **18**: Gewindeflanke
- **19**: oberer Umfangsrand
- **20**: Axialabschnitt
- **21**: Ende
- **22**: Innengewinde
- **23**: Durchgangsöffnung
- **24**: Knochenplatte
- **25**: Innengewinde für Flanke
- **26**: System

- **L**: Längsmittelachse der Knochenschraube
- **L_{D}**: Längsmittelachse der Durchgangsöffnung
- **α**: Winkel zwischen Freistichflanke und Querebene
- **β**: Winkel zwischen Längsmittelachse und Mantelfläche der konischen Hüllkontur des Axialabschnitts
- **Y**: Winkel zwischen L und L_{D}

## Patentansprüche

1. Knochenschraube mit einem ein Knochengewinde (3) aufweisenden Schaft (2) und mit einem axial benachbart zum Knochengewinde (3) angeordneten, Gewinderillen aufweisenden Gewinde (7), wobei das Gewinde (7) in einem eine konische Hüllkontur aufweisenden Axialabschnitt (8) eines Schraubenkopfes (6) eingebracht ist, welcher den Schaft (2) nach außen überragt, wobei eine Steigung des Knochengewindes (3) und eine Steigung des Gewindes (7) gleich sind,
**dadurch gekennzeichnet,**
**dass** das Gewinde (7) mit einem vom Knochengewinde (3) abgewandten Ende in einen umlaufenden Freistich (11) ausläuft, der von einer zur Verblockung mit einer Knochenplatte bestimmten, als Schrägebene ausgebildeten oberen Freistichflanke (12) begrenzt ist, die sich bis zum Gewinderillengrund (9) des in den Freistich (11) auslaufenden Gewindes (7) erstreckt.

2. Knochenschraube nach Anspruch 1,
**dadurch gekennzeichnet,**
**dass** ein Winkel (α) zwischen einer quer zu einer Längsmittelachse angeordneten, einen Freistichgrund aufnehmenden Freistichebene (e) und der oberen Freistichflanke (12) aus einem Wertebereich zwischen etwa 10° und etwa 50°, vorzugsweise zwischen etwa 15° und etwa 45°, bevorzugt zwischen etwa 20° und etwa 35°, gewählt ist oder etwa 30° beträgt.

3. Knochenschraube nach einem der vorhergehenden Ansprüche,
**dadurch gekennzeichnet,**
**dass** die Gewinderillen (10) radial innen auf einer gedachten Zylindermantelfläche oder auf einer gedachten Konusmantelfläche enden.

4. Knochenschraube nach einem der vorhergehenden Ansprüche,
**dadurch gekennzeichnet,**
**dass** das Gewinde (7) axial von dem Knochengewinde (3) beabstandet ist, vorzugsweise derart, dass die Gewinderillen des Gewindes (7) und das Knochengewinde (3) konzentrisch zu einer gemeinsamen, gedachten Schraubenlinie einheitlicher Steigung angeordnet sind.

5. Knochenschraube nach einem der vorhergehenden Ansprüche,
**dadurch gekennzeichnet,**
**dass** das umlaufende Gewinde (7) ein mehrgängiges, insbesondere dreigängiges, und/oder ein metrisches Gewinde (7) ist.

6. System, umfassend eine mindestens eine Durchgangsöffnung (23), insbesondere mit Innengewinde (22), aufweisende Knochenplatte (24) und eine Knochenschraube (1) nach einem der vorhergehenden Ansprüche, wobei die Freistichflanke (12) mit der Knochenplatte (24) verblockbar ist.

7. System nach Anspruch 6,
**dadurch gekennzeichnet,**
**dass** die Knochenschraube (1) aus einem härteren Material als die Knochenplatte (24) ausgebildet ist.

8. System nach einem der Ansprüche 6 oder 7,
**dadurch gekennzeichnet,**
**dass** die Radialerstreckung der oberen Freistichflanke (12) größer ist als der Kerndurchmesser der Durchgangsöffnung (23).

## Claims

1. Bone screw comprising a shank (2) having a bone thread (3), and comprising a thread (7) which is arranged axially adjacent to the bone thread (3) and has thread grooves, the thread (7) being introduced into an axial portion (8) of a screw head (6), which protrudes outwards above the shank (2) which portion has a conical enveloping contour, a gradient of the bone thread (3) and a gradient of the thread (7) being equal, **characterised in that** the thread (7) tapers off at an end remote from the bone thread (3) into a circumferential undercut (11) which is limited by an upper undercut flank (12) which is intended to lock with a bone plate, is formed as an incline plane, and extends up to the thread groove base (9) of the thread (7) which tapers off into the undercut (11).

2. Bone screw according to claim 1, **characterised in that** an angle (α) between an undercut plane (e), which is arranged transverse to a central longitudinal axis and receives an undercut base, and the upper undercut flank (12) is selected from a range of values between approximately 10° and approximately 50°, preferably between approximately 15° and approximately 45°, particularly preferably between approximately 20° and approximately 35° or is approximately 30°.

3. Bone screw according to any of the preceding claims, **characterised in that** the thread grooves (10) end radially inwardly on an imaginary cylindrical generated surface or on an imaginary conical generated surface.

4. Bone screw according to any of the preceding claims, **characterised in that** the thread (7) is axially spaced from the bone thread (3), preferably such that the thread grooves of the thread (7) and the bone thread (3) are arranged concentrically with a common imaginary helix having a uniform gradient.

5. Bone screw according to any of the preceding claims, **characterised in that** the circumferential thread (7) is a multi-start, in particular a triple-start, and/or a metric thread (7).

6. System comprising a bone plate (24) having at least one through-opening (23), in particular having an internal thread (22), and a bone screw (1) according to any of the preceding claims, wherein the undercut flank (12) can be locked with the bone plate (24).

7. System according to claim 6, **characterised in that** the bone screw (1) is formed from a harder material than the bone plate (24).

8. System according to either claim 6 or claim 7, **characterised in that** the radial extension of the upper undercut flank (12) is greater than the core diameter of the through-opening (23).

## Revendications

1. Vis à os comprenant une tige (2) présentant un filetage pour os (3) et comprenant un filetage (7) présentant des rainures de filet disposées axialement à côté du filetage pour os (3), le filetage (7) étant pratiqué dans une portion axiale (8) d'une tête de vis (6) présentant un contour d'enveloppe conique, laquelle tête de vis dépasse vers l'extérieur au-delà de la tige (2), un pas du filetage pour os (3) et un pas du filetage (7) étant identiques,
**caractérisée en ce que**
le filetage (7) se termine avec une extrémité opposée au filetage pour os (3) dans un dégagement par rainure périphérique (11) qui est limité par un flanc de dégagement supérieur (12) prévu pour réaliser un blocage avec une plaque à os, réalisé sous forme de plan oblique, qui s'étend jusqu'au fond de rainure de filet (9) du filetage (7) se terminant dans le dégagement par rainure (11).

2. Vis à os selon la revendication 1,
**caractérisée en ce**
**qu'**un angle (α) entre un plan de dégagement par rainure (e) recevant un fond de dégagement par rainure, disposé transversalement à un axe médian longitudinal, et le flanc de dégagement supérieur (12), est choisi dans une plage de valeurs comprise entre environ 10° et environ 50°, de préférence entre environ 15° et environ 45°, de préférence entre environ 20° et environ 35°, ou vaut environ 30°.

3. Vis à os selon l'une quelconque des revendications précédentes,
**caractérisée en ce que**
les rainures de filet (10) se terminent radialement à l'intérieur sur une surface d'enveloppe cylindrique imaginaire ou sur une surface d'enveloppe conique imaginaire.

4. Vis à os selon l'une quelconque des revendications précédentes,
**caractérisée en ce que**
le filetage (7) est espacé axialement du filetage pour os (3), de préférence de telle sorte que les rainures de filetage du filetage (7) et le filetage pour os (3) soient disposés concentriquement par rapport à une ligne hélicoïdale commune imaginaire de pas uniforme.

5. Vis à os selon l'une quelconque des revendications précédentes,
**caractérisée en ce que**
le filetage périphérique (7) est un filetage (7) à plusieurs pas, notamment à trois pas, et/ou un filetage métrique.

6. Système comprenant une plaque à os (24) présentant au moins une ouverture de passage (23), en particulier avec un filetage interne (22), et une vis à os (1) selon l'une quelconque des revendications précédentes, le flanc de dégagement (12) pouvant être bloqué avec la plaque à os (24).

7. Système selon la revendication 6,
**caractérisé en ce que**
la vis à os (1) est réalisée à partir d'un matériau plus dur que la plaque à os (24).

8. Système selon l'une quelconque des revendications 6 ou 7,
**caractérisé en ce que**
l'étendue radiale du flanc de dégagement supérieur (12) est supérieure au diamètre du coeur de l'ouverture de passage (23).
